# EUROPÄISCHE PATENTSCHRIFT

(11) **EP 0 581 915 B1**
(45) Veröffentlichungstag und Bekanntmachung des Hinweises auf die Patenterteilung: **07.02.1996**
(21) Anmeldenummer: 92924493.7
(22) Anmeldetag: 14.12.1992
(51) Int. Cl.: G01N 17/02, G01N 33/38

(54) **ELEKTRODE ZUR FESTSTELLUNG DES KORROSIONSZUSTANDES DER METALLBEWEHRUNG VON BETONBAUWERKEN**
ELECTRODE FOR DETERMINING THE STATE OF CORROSION OF METAL REINFORCEMENT IN CONCRETE CONSTRUCTIONS
ELECTRODE SERVANT A DETERMINER LE DEGRE DE CORROSION DE L'ARMATURE METALLIQUE D'OUVRAGES EN BETON

(30) Priorität: 21.02.1992 AT 321/92; 20.11.1992 AT 2307/92
(43) Veröffentlichungstag der Anmeldung: 09.02.1994
(73) Patentinhaber: WIETEK, Bernhard, A-6073 Sistrans Nr. 290 (AT)
(72) Erfinder: WIETEK, Bernhard, A-6073 Sistrans Nr. 290 (AT)
(74) Vertreter: Hofinger, Engelbert, DDr.
(86) Internationale Anmeldenummer: AT9200167
(87) Internationale Veröffentlichungsnummer: WO9317323

(56) Entgegenhaltungen:
- EP-A- 0 364 841
- GB-A- 2 169 410
- GB-A- 2 180 069

## Beschreibung

Die Erfindung bezieht sich auf eine Elektrode zur Feststellung des Korrosionszustandes der Metallbewehrung von Betonbauwerken, in Form eines gegenüber der Bewehrung elektrochemisch höherwertigen, von einer Isolierung umgebenen Drahtes, dessen elektrisch aktiver Bereich mit der Feuchtigkeit im Beton in elektrolytischer Verbindung steht.

Grundsätzlich ist ein Korrosionsschutz der Bewehrung von Betonbauwerken bereits durch die hohe Alkalität des Betons verursacht, durch welche sich auf der Oberfläche des Bewehrungsstahls eine rosthemmende Passivschicht bildet. Soferne trotzdem in den vergangenen Jahren Korrosionsschäden bei Stahlbetonbauwerken festgestellt worden sind, handelt es sich überwiegend um Verkehrsbauwerke, wie Brücken, und die überwiegende Schadensursache war die Einwirkung von Streusalz. Außer durch Salze kann die Passivschicht auf der Stahloberfläche aber beispielsweise auch dadurch zerstört werden, daß der Beton mit dem Kohlendioxid der umgebenden Luft reagiert und somit durch Karbonatisierung seine Alkalität verliert.

Mit Hilfe von Elektroden läßt sich jenes Potential messen, das sich an der Grenzfläche zwischen Stahl und Beton einstellt. An der ordnungsgemäß hergestellten und eingebauten Bewehrung mit unbeschädigtem Korrosionsschutz stellt sich mit der Zeit ein ständiges Potential ein, das dem passivierten Zustand der Bewehrung entspricht (Bereich der Passivität). Bei einer Änderung im Zustand des Zementmörtels, die zur Korrosion führen kann, wird eine Abnahme des Potentials festgestellt. Am Bewehrungsstahl mit korrodierenden Stellen ist diese Abnahme besonders groß.

Eine noch zuverlässigere Abschätzung der Korrosionswahrscheinlichkeit kann durch die Potentialmessung nach einer anodischen Polarisation erzielt werden. Über eine der Meßelektroden wird ein kurzzeitiger anodischer galvanostatischer Gleichstromimpuls auf die Bewehrung gegeben und die sich einstellende Änderung des Potentials gemessen. In einer korrosionsfreien Zone ergibt sich eine größere Potentialänderung als in einer Zone mit Korrosionsstellen.

In DD 72398 ist eine Elektrode der eingangs skizzierten Art beschrieben worden, bei welcher der elektrisch aktive Bereich frei im Beton liegt und nur der von diesem nach außen führende Verbindungsdraht von einer geschlossenen Isolierung umgeben ist. Es ist jedoch schwierig, so wie in DD 72398 vorgeschrieben, die zur Messung dienende Hilfselektrode parallel in definiertem Abstand vom Bewehrungsstahl einzubauen. Bereits bei nachträglich gespannten Litzenankern ist es schwierig, metallischen Kontakt zwischen der Elektrode und der Bewehrung zuverlässig zu vermeiden. Punktweiser Kontakt zwischen Elektrode und Bewehrung oder eine extreme, das Ergebnis verfälschende Annäherung dieser beiden Elemente aneinander ist natürlich erst recht zu befürchten, wollte man die Meßelektrode zusammen mit einer beliebigen Bewehrung in den Beton einbringen.

Aufgabe der Erfindung ist es, das Anwendungsgebiet der elektrischen Überwachung von Bewehrungen auf Korrosionsfreiheit zu erweitern, indem Überwachungselektroden in beliebig bewehrte Bauwerke ohne besondere Anforderungen an die Genauigkeit ihrer Positionierung eingebracht werden. Erfindungsgemäß wird zur Lösung dieser Aufgabe vorgeschlagen, daß die Isolierung entlang des gesamten elektrisch aktiven Bereiches des Drahtes so angeordnet ist, daß sie diesen Bereich im Abstand von der Bewehrung hält und von dieser galvanisch isoliert.

Die elektrischen Eigenschaften des Materials der Isolierung sollen jenen des Betons möglichst gleichen, weshalb es durchaus möglich wäre, den Draht vor seiner Einbringung in den Ortbeton mit kleinen Betonkörpern zu umgeben. Aus Gründen der leichteren Herstellbarkeit und mechanischen Festigkeit empfiehlt es sich jedoch, ein anderes Material zu nehmen, welches ebenfalls galvanisch nicht leitet, für die als Elektrolyt wirkende Feuchtigkeit im Beton jedoch durchlässig ist. Insbesondere kommt hier Ton in Frage, welcher zur Erhaltung seiner Porosität bei relativ niedrigen Temperaturen (beispielsweise 850°C) gebrannt ist. Auch Pappe weist die geforderten elektrischen Eigenschaften auf.

Chemisch sollen die verwendeten Isolierkörper möglichst neutral sein, jedenfalls soll ihre eigene elektrochemische Aktivität das Ergebnis nicht verfälschen.

Wenn die Isolierkörper sich in ihren elektrischen Eigenschaften von Beton nur unwesentlich unterscheiden, sind sie auf das Meßergebnis praktisch ohne Einfluß. Selbst ein geringer systematischer Einfluß der Isolierkörper ist jedoch unschädlich, da die Korrosionsüberwachung ja nicht auf dem Absolutwert des gemessenen Potentials, sondern auf der korrosionsabhängigen Änderung desselben aufbaut.

Aus diesem Grunde ist es auch möglich, den Isolierkörper aus einem Material zu formen, welches selbst nicht für die Feuchtigkeit im Bauwerk durchlässig ist. Voraussetzung ist, daß diese Isolierung genügend große Zwischenräume freiläßt, durch welche der Beton direkt mit dem blanken Draht der Elektrode in Berührung kommt. Bei einer derartigen Ausführung ist es besonders günstig, wenn die Isolierung aus übereinander gegenläufig um den Draht gewickelten Litzen aus isolierendem Material, insbesondere aus Kunststoff, besteht.

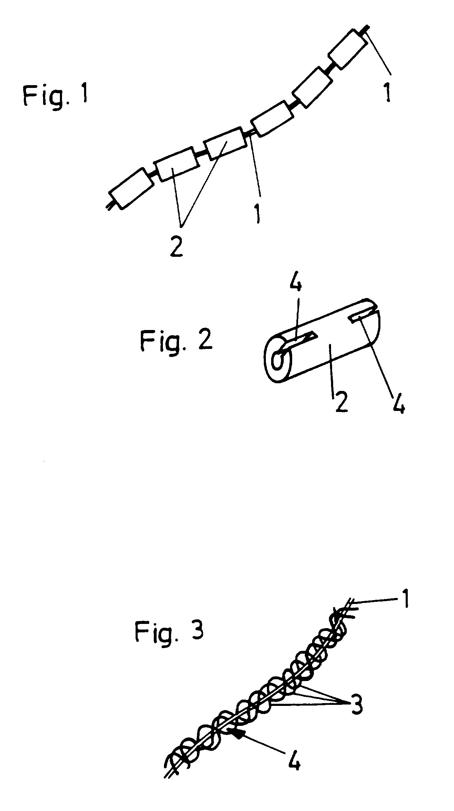

Anschließend werden anhand der Zeichnung einige Ausführungsformen der Erfindung dargestellt, wobei Fig. 1 - 3 sich jeweils auf eines von insgesamt drei Ausführungsbeispielen bezieht.

Beim Ausführungsbeispiel nach Fig. 1 ist ein Draht 1 in zumindest jenem Teil, welcher im Inneren eines Betonbauwerkes als aktiver Teil einer Elektrode wirken soll, von Perlen 2 umgeben, welche als Kugeln, Zylinder od. dgl. ausgebildet sein können. Den geringsten Einfluß auf das Meßergebnis haben diese Perlen 2, wenn sie aus porösem Material bestehen, dessen Eigenschaften jenem von Beton möglichst nahekommen.

Wie Fig. 2 zeigt, ist es auch möglich, die auf den Draht 1 aufgefädelten Isolierkörper aus undurchlässigem Material, beispielsweise aus Kunststoff, zu formen, wenn darin genügend große Zwischenräume 4 vorgesehen sind, durch welche der Beton bis an den blanken Draht 1 dringen kann.

Einfacher als das Auffädeln von perlenartigen Isolierkörpern auf den Draht ist das Umwickeln desselben mit gegenläufigen Gängen von Fäden 3 aus isolierendem Material, wie dies in der Darstellung nach Fig. 3 angedeutet ist. In die Zwischenräume 4 zwischen den Fäden 3 kann der Beton leicht eindringen, wogegen andererseits Fäden 3 als Abstandhalter zur nicht dargestellten Bewehrung dienen.

Eine derartige Elektrode kann mit der zu überwachenden Bewehrung, beispielsweise einem Baustahlgitter oder einem Anker, mit Ortbeton umgossen werden, wobei ein Ende der Elektrode von außen kontaktierbar sein muß. Dies gilt natürlich auch für die Bewehrung selbst.

Falls beabsichtigt ist, durch die Elektrode einen bestimmten Teil der Bewehrung, beispielsweise einen Anker, zu überwachen, ist es möglich, die Elektrode und den Bewehrungsteil mit einer gemeinsamen Isolierung, beispielsweise einem Hüllrohr, zu umgeben, wie dies bereits in DE-A1-40 10 800 vorgeschlagen worden ist.

## Patentansprüche

1. Elektrode zur Feststellung des Korrosionszustandes der Metallbewehrung von Betonbauwerken, in Form eines gegenüber der Bewehrung elektrochemisch höherwertigen, von einer Isolierung umgebenen Drahtes, dessen elektrisch aktiver Bereich mit der Feuchtigkeit im Beton in elektrolytischer Verbindung steht, dadurch gekennzeichnet, daß die Isolierung entlang des gesamten elektrisch aktiven Bereiches des Drahtes so angeordnet ist, daß sie diesen Bereich im Abstand von der Bewehrung hält und von dieser galvanisch isoliert.

2. Elektrode nach Anspruch 1, dadurch gekennzeichnet, daß die Isolierung aus porösem Material, insbesondere aus Ton oder Pappe besteht.

3. Elektrode nach Anspruch 1 oder 2, dadurch gekennzeichnet, daß die Isolierung Zwischenräume (4) freiläßt, wobei der in diese Zwischenräume (4) eingedrungene Beton direkt mit dem Draht (1) in Berührung steht.

4. Elektrode nach einem der Ansprüche 1 bis 3, dadurch gekennzeichnet, daß die Isolierung aus auf den Draht (1) aufgefädelten Perlen, insbesondere aus gebranntem Ton oder Pappe, besteht.

5. Elektrode nach einem der Ansprüche 1 bis 3, dadurch gekennzeichnet, daß die Isolierung aus übereinander gegenläufig um den Draht (1) gewickelten Litzen (3) aus isolierendem Material, insbesondere aus Kunststoff, besteht.

## Claims

1. Electrode for determining the state of corrosion of metal reinforcement in concrete constructions, in the form of a wire that is more electro-positive than the reinforcement and surrounded by insulation, the electrically active part of said wire being in electrolytic contact with the moisture in the concrete, characterized in that the insulation is so arranged along the whole electrically active part of the wire that it keeps this part at a distance from the reinforcement and insulates it from this galvanically.

2. Electrode as defined in claim 1, characterized in that the insulation consists of porous material, in particular of clay or cardboard.

3. Electrode as defined in claim 1 or 2, characterized in that the insulation leaves open intervening spaces (4) such that the concrete that has penetrated into these intervening spaces (4) is in direct contact with the wire (1) .

4. Electrode as defined in one of the claims 1 to 3, characterized in that the insulation consists of beads, in particular of fired clay or cardboard, that are threaded onto the wire (1).

5. Electrode as defined in one of the claims 1 to 3, characterized in that the insulation consists of threads (3) of insulating material, in particular of plastic, that are wound onto the wire (1) over each other and in opposite directions.

## Revendications

1. Electrode pour déterminer l'état de corrosion de l'armature métallique de constructions en béton, en forme de fil qui a une valence supérieure à celle de l'armature, qui est entouré d'une isolation et dont la zone électriquement active est en liaison électrolytique avec l'humidité du béton, caractérisée en ce que l'isolation est disposée le long de toute la zone électriquement active du fil de manière à maintenir cette zone espacée de l'armature et à l'isoler électriquement de ladite armature.

2. Electrode selon la revendication 1, caractérisée en ce que l'isolation se compose d'un matériau poreux, en particulier d'argile ou de carton.

3. Electrode selon la revendication 1 ou 2, caractérisée en ce que l'isolation laisse des espaces intermédiaires (4), le béton entré dans ces espaces intermédiaires (4) étant en contact direct avec le fil (1).

4. Electrode selon l'une des revendications 1 à 3, caractérisée en ce que l'isolation se compose de perles, en particulier en argile cuite ou en carton, enfilées sur le fil (1).

5. Electrode selon l'une des revendications 1 à 3, caractérisée en ce que l'isolation se compose de torons (3) en matériau isolant, notamment en matière plastique, enroulés sur le fil (1) les uns au-dessus des autres et en sens inverse.
